# EUROPÄISCHE PATENTSCHRIFT

(11) **EP 3 157 604 B1**
(45) Veröffentlichungstag und Bekanntmachung des Hinweises auf die Patenterteilung: **15.08.2018**
(21) Anmeldenummer: 15730060.9
(22) Anmeldetag: 02.06.2015
(51) Int. Cl.: A61M 16/18, A61M 16/10

(54) **VORRICHTUNG UND VERFAHREN ZUR DOSIERUNG EINES NARKOSEMITTELS IN EINEN GASSTROM**
DEVICE AND METHOD FOR DOSING AN ANAESTHETIC IN A GAS FLOW
DISPOSITIF ET PROCÉDÉ DE DOSAGE D'UN ANESTHÉSIANT DANS UN FLUX DE GAZ

(30) Priorität: 17.06.2014 DE 102014008625
(43) Veröffentlichungstag der Anmeldung: 26.04.2017
(73) Patentinhaber: Drägerwerk AG & Co. KGaA, 23558 Lübeck (DE)
(72) Erfinder: WRUCK, Norbert, 23568 Lübeck (DE)
(86) Internationale Anmeldenummer: PCT/EP2015/001122
(87) Internationale Veröffentlichungsnummer: WO 2015/192946

(56) Entgegenhaltungen:
- DE-A1- 3 234 474
- DE-A1- 4 105 858

## Beschreibung

Die Erfindung betrifft eine Vorrichtung sowie ein Verfahren zur Dosierung eines Narkosemittels in einen Gasstrom gemäß den Oberbegriffen der unabhängigen Patentansprüche. Eine gattungsgemäße Dosiervorrichtung weist zwischen ihrem Gaseinlass und dem Gasauslass eine Verdunstungskammer auf, in der dem Gasstrom zumindest zeitweise ein Narkosemittel zudosierbar ist, und verfügt ferner über ein Heizelement, das zumindest zeitweise das Gas erwärmt.

Aus dem Stand der Technik ist eine Vielzahl von Anästhesiegeräten bekannt, mit denen einem Atemgasstrom für einen Patienten ein Narkosemittel zur zeitweisen Anästhesierung des in diesem Zeitraum durch das Anästhesiegerät beatmeten Patienten zugeführt wird. Hierbei kommen für die Anreicherung des Atemgasstroms mit volatilen Narkosemitteln nach wie vor überwiegend mechanisch arbeitende Narkosemittelverdunster zum Einsatz. Derartige Geräte, die nach dem Stromteilerprinzip arbeiten, zeichnen sich durch ihre Zuverlässigkeit aus und sind daher weithin akzeptiert. Problematisch an diesen Geräten ist allerdings, dass vergleichsweise große thermische Speichermassen verwendet werden müssen, um zu verhindern, dass beim Abfordern von großen Verdampfungsmassenströmen das Gerät auskühlt, und es so zu einem unzulässigen Absinken des Dampfdrucks und damit der abzugebenden Dampfleistung kommen würde. Aufgrund der benötigten thermischen Speichermassen sind mechanisch arbeitende Narkosemittelverdunster oftmals verhältnismäßig schwer, was wiederum nachteilig für die Handhabung ist.

Alternativ zu den vorgenannten Narkosemittelverdampfern sind leistungsstarke elektronische Verdampfer bekannt, wie sie etwa in der DE 31 16 951 A1 beschrieben werden. Diese Narkosemittelverdampfer, die über eine hohe Dampfabgabeleistung verfügen, arbeiten mit unter Druck gesetztem Narkosemittel und erfordern somit eine vergleichsweise aufwändige Sicherheitstechnik. Um eine ausreichende Sicherheit der Geräte zu gewährleisten, ist daher eine Vielzahl von Sensorik- und Aktorikbauteilen erforderlich, was sich wiederum negativ auf die Kosten für derartige Geräte und deren Ausfallrate auswirkt.

Ferner ist aus der WO 2004/091708 ein Narkosemittelverdunster bekannt, bei dem mithilfe eines geregelt betriebenen Proportionalventils der Narkosemittelfluss bedarfsgerecht eingestellt wird. Hierbei basiert die Regelung auf einer Frischgasmessung und einem geschlossenen Regelkreis auf der Flüssigseite. Der beschriebene Narkosemittelverdunster verfügt über eine Verdunstungskammer, die an den Frischgasstrom angebunden ist, wobei innerhalb der Verdunstungskammer ein Heizelement vorgesehen ist, durch das ein bedarfsgerechtes Verdunsten des flüssigen Narkosemittels gewährleistet wird. Nachteilig an diesem vollelektronisch arbeitenden System ist der hohe Aufwand für die benötigte Sensorik bzw. Aktorik. Aus der DE 32 34 474 ist eine Vorrichtung zur Dosierung eines Narkosemittels bekannt, die die Merkmale des Oberbegriffs des Anspruchs 1 aufweist.

Ausgehend von den bekannten technischen Lösungen zur Dosierung eines Narkosemittels in einem Atemgasstrom liegt der Erfindung die Aufgabe zugrunde, ein entsprechendes System derart weiterzubilden, dass einerseits die Abgabe selbst großer Dampfmengen möglich ist und andererseits der konstruktive und regelungstechnische Aufwand vergleichsweise gering ist. Die anzugebende Vorrichtung soll insbesondere nur über wenig Sensortechnik sowie Stellelemente verfügen, so dass eine leichte, ausfallsichere, kostengünstige und dennoch leistungsstarke Narkosemitteldosierung realisiert wird. Wesentlich hierbei ist, dass das Ziel erreicht wird, ohne dass die verwendeten volatilen Narkosemittel einer unzulässigen thermischen Belastung ausgesetzt werden. Gleichzeitig sollen die sicherheitstechnischen und wirtschaftlichen Nachteile von druckbeaufschlagten Systemen zur Narkosemitteldosierung ausgeschlossen werden.

Die vorstehende Aufgabe wird mit einer Vorrichtung gemäß Anspruch 1 sowie einem Verfahren nach Anspruch 11 gelöst. Vorteilhafte Ausführungsformen der Erfindung sind Gegenstand der abhängigen Ansprüche und werden in der folgenden Beschreibung unter teilweiser Bezugnahme auf die Figuren näher erläutert.

Die Erfindung betrifft eine Vorrichtung zur Dosierung eines Narkosemittels in einen Gasstrom, in die über einen Gaseinlass ein dem Narkosemittel anzureicherndes Gas von einer Atemgasquelle kommend einströmt und aus der über einen Gasauslass das mit dem Narkosemittel angereicherte Gas zu einem Patientenanschluss ausströmt. In Strömungsrichtung des Gases zwischen dem Gaseinlass und dem Gasauslass sind eine Verdunstungskammer, in der dem Gasstrom zumindest zeitweise ein Narkosemittel zudosiert wird, und ein Heizelement zur zumindest zeitweisen Erwärmung des Gases angeordnet. Erfindungsgemäß ist diese Vorrichtung derart weitergebildet worden, dass das Heizelement in Strömungsrichtung des Gases vor der Verdunstungskammer angeordnet ist und von einer Steuereinheit derart ansteuerbar ist, dass dem mit Narkosemittel anzureichernden Gas Wärme zur Verdunstung des in der Verdunstungskammer befindlichen Narkosemittels in Abhängigkeit wenigstens eines Zustandsparameters des mit dem Narkosemittel anzureichernden und/oder des zumindest teilweise mit dem Narkosemittel angereicherten Gases zugeführt wird. Der wesentliche Gedanke der Erfindung beruht somit auf einer neuartigen Ausführung des passiven Verdunsterprinzips, wobei die für die Verdunstung des im Narkosemittelverdunster befindlichen Narkosemittels benötigte Wärme bedarfsgerecht, quasi mittelbar in die Verdunstungskammer eingebracht wird, und zwar über den in die Verdunstungskammer eintretenden und mit dem Narkosemittel anzureichernden Gasstrom. Die Einbringung der notwendigen Wärme in den mit Narkosemittel anzureichernden Gasstrom findet hierbei in Abhängigkeit wenigstens eines Zustandsparameters bzw. einer thermodynamischen Zustandsgröße des in Strömungsrichtung vor, in und/oder hinter der Verdunstungskammer befindlichen Gasstroms. Vorzugsweise erfolgt die Steuerung der Heizleistung unter Berücksichtigung einer Temperatur und/oder einer stofflichen Zusammensetzung des Gasstroms. Auf besonders bevorzugte Weise wird hierbei ein geschlossener Regelkreis erzeugt, wobei ein einstellbarer Wert für den wenigstens einen Zustandsparameter des vor, in und/oder hinter der Verdunstungskammer befindlichen Gasstroms die Führungsgröße und die jeweils notwendige Heizleistung die Steuergröße darstellt.

In einer besonderen Ausführungsform der Erfindung erfolgt die Ansteuerung des Heizelements in Abhängigkeit einer nach Verlassen der Verdunstungskammer benötigten Narkosemittelmenge. In diesem Fall wird dem mit Narkosemittel anzureichernden Gasstrom vor Eintritt in die Verdunstungskammer so viel Wärme zugeführt, dass ein mit Narkosemittel angereicherter Gasstrom die Verdunstungskammer verlässt, in dem das Narkosemittel eine vorgegebene bzw. gewünschte Narkosemittelkonzentration bzw. -menge aufweist.

Gemäß einer speziellen Ausführungsform der Erfindung ist das Heizelement derart ansteuerbar, dass dem mit Narkosemittel anzureichernden Gas Wärme zur Verdunstung des in der Verdunstungskammer befindlichen Narkosemittels in Abhängigkeit einer nach Verlassen der Verdunstungskammer benötigten Sollkonzentration des Narkosemittels im Gas und/oder eines erforderlichen Narkosemittelvolumenstroms zugeführt wird. Vorzugsweise erfolgt die Ansteuerung des Heizelementes hierbei unter Berücksichtigung eines Gasvolumenstroms des im Dosierkanal vor und/oder hinter der Verdunstungskammer strömenden Gasstroms.

Gemäß einer besonderen Weiterbildung wird aus dem jeweiligen Gasvolumenstrom, der erforderlichen Soll-Konzentration und/oder des erforderlichen Narkosemittelvolumenstroms sowie der thermodynamischen Stoffwerte des volatilen Narkosemittels, wie beispielsweise der spezifischen Verdampfungsenthalpie und der spezifischen Wärmekapazität, ermittelt, welcher Wärmestrom der Verdunstungskammer im jeweiligen Betriebszustand zugeführt werden muss. Die Steuerung basiert somit auf einer energieverfahrenstechnischen Bilanzierung hinsichtlich Masse und Enthalpie. Das mit dem Narkosemittel anzureichernde Gas kommt vorzugsweise aus einem Gasmischer einer Anästhesiemaschine, wobei der verwendete Gasmischer auf vorteilhafte Weise elektronisch geregelt ist. In einer besonderen Ausführungsform wird dem in die Verdunstungskammer einzuleitenden Gasstrom so viel Wärme zugeführt, dass der die Verdunstungskammer verlassende Gasstrom mit Narkosemittel gesättigt ist. Eine nachträgliche Reduzierung der Narkosemittelkonzentration wird in diesem Fall bei Bedarf durch eine geeignete Beimischung von Frischgas erreicht.

Wesentlich ist in jedem Fall, dass durch Erwärmen des mit Narkosemittel anzureichernden Gasstroms und die anschließende in der Verdunstungskammer selbstständig ablaufende Abkühlung während des Verdunstungsprozesses dem im Verdunster befindlichen flüssigen, volatilen Narkosemittel die Verdampfungsenthalpie bedarfsgerecht zugeführt wird. Die Zufuhr der im jeweiligen Zustand benötigten Wärme erfolgt dabei fluidgetragen, also mithilfe des erwärmten Gases, das aus einer Gasquelle entnommen wurde. Ein besonderer Vorteil dieser Form der Wärmezuführung besteht darin, dass die Verdunstungskammer vergleichsweise leicht ausgeführt werden kann, da diese im Gegensatz zu den aus dem Stand der Technik bekannten Lösungen nur geringe thermische Massen aufweisen muss.

Gemäß der Erfindung weist die Verdunstungskammer einen Tank für flüssiges 'Narkosemittel und einen Verdunstungsbereich, in dem flüssiges Narkosemittel wenigstens teilweise während des Betriebs verdunstet, auf. Vorzugsweise sind der Tank und der Verdunstungsbereich als ein Bauteil ausgeführt, das als Narkosemittelverdunster bezeichnet werden kann. In diesem Zusammenhang ist es auf bevorzugte Weise denkbar, dass ein derartiges Bauteil bzw. Narkosemittelverdunster über wenigstens ein geeignetes Befestigungselement zur lösbaren Verbindung mit einem Anästhesiegerät verfügt. Das Vorsehen eines derartigen Befestigungselementes stellt sicher, dass ein Narkosemittelverdunster vergleichsweise einfach, insbesondere zur Reinigung, Wartung oder Befüllung mit Narkosemittel, von einer Anästhesiemaschine abnehmbar und nach Beendigung der durchgeführten Arbeiten wieder an dieser befestigbar ist. Selbstverständlich ist es hierbei ebenfalls auf einfache Weise möglich, einen entsprechend ausgerüsteten Narkosemittelverdunster durch einen baugleich ausgeführten zu ersetzen.

Weiterhin ist in einer speziellen Ausgestaltung vorgesehen, das Heizelement, das strömungstechnisch der Verdunstungskammer vorgeschaltet ist, gemeinsam mit der Verdunstungskammer als konstruktiv einheitliches Bauelement auszuführen. Vorzugsweise ist hierfür eine Außenwand der Verdunstungskammer derart geformt, dass das Heizelement innerhalb der von der Außenwand gebildeten Ausnehmung, die bevorzugt halboffen ist, aufgenommen wird. Auf vorteilhafte Weise verfügt das Heizelement über wenigstens ein Befestigungselement, mit dem dieses zumindest mittelbar an der Außenwand der Verdunstungskammer befestigbar ist.

In einer besonderen Weiterbildung der Erfindung sind das Heizelement und die Verdunstungskammer strömungstechnisch parallel zu einem Frischgaskanal angeordnet, wobei die beiden einerseits den Frischgaskanal und andererseits die Verdunstungskammer durchströmenden Teilgasströme vor einem Patientenanschlussstück in einem Mischpunkt zusammen geführt werden. Gemäß dieser Ausführungsform sind somit einerseits ein Dosierkanal, in dem das Heizelement und die Verdunstungskammer in Reihe angeordnet sind, und andererseits ein strömungstechnisch parallel zum Dosierkanal angeordneter Frischgaskanal vorgesehen, durch die die beiden Teilgasströme bedarfsgerecht geleitet werden.

Gemäß einer weiteren Ausführungsform ist es denkbar, dass das Heizelement derart angesteuert wird, dass eine Temperatur einer den Verdunstungsbereich der Verdunstungskammer wenigstens bereichsweise begrenzenden Kammerwand während des Betriebs zumindest nahezu konstant bleibt. Im stationären Betriebszustand erfolgt somit eine bedarfsgerechte Bestimmung der Temperaturdifferenz zwischen dem in die Verdunstungskammer einströmenden Gasstrom und der dort befindlichen Narkosemittelflüssigkeit. Für die Steuerung des Heizelementes, also zur Ermittlung der in der Verdunstungskammer benötigten Wärme, werden die Menge, insbesondere der Volumenstrom, sowie die Temperatur des mit dem Narkosemittel anzureichernden Gasstroms berücksichtigt. Hierbei wird durch konstruktive Maßnahmen und/oder eine gezielte Materialauswahl die Zufuhr von in der Verdunstungskammer bzw in deren Bauteilen gespeicherter Wärme begrenzt, so dass die Verdunstung des für die Anreicherung des Gasstroms benötigten Narkosemittels in der Verdunstungskammer unter quasi-adiabaten Zustandsbedingungen, insbesondere im Bereich von Oberflächen erfolgt. Vorzugsweise erfolgt die Verdunstung des Narkosemittels in der Verdunstungskammer nach dem Prinzip einer zumindest nahezu adiabaten Oberfläche.

Die Zufuhr von Wärme erfolgt somit idealerweise ausschließlich über die Erwärmung des mit Narkosemittel anzureichernden Gasstroms vor dessen Eintritt in die Verdunstungskammer. Die thermische Masse der Verdunstungskammer, insbesondere der den Verdunstungsbereich umgebenden Bauteile, wird somit deutlich begrenzt und daher gleichzeitig die Masse der Verdunstungskammer gegenüber bekannten Narkosemittelverdunstern erheblich verringert. Ein unzulässiges Auskühlen des Narkosemittelverdunsters wird gemäß der zuvor beschriebenen speziellen Ausführungsform der Erfindung durch ein entsprechendes Aufheizen des Heizelementes und somit eine zusätzliche Erwärmung des mit Narkosemittel anzureichernden Gasstroms erreicht.

Bei einer besonderen Weiterbildung der Erfindung ist in Strömungsrichtung hinter der Verdunstungskammer ein Temperatursensor zur Erfassung einer Temperatur des mit Narkosemittel angereicherten Gases vorgesehen, wobei der Sensor über eine Datenstrecke mit der Steuereinheit verbunden ist. Eine entsprechende zur Datenübertragung geeignete Datenstrecke kann in Abhängigkeit des Anwendungsfalls drahtgebunden oder drahtlos ausgeführt sein. Vorzugsweise befindet sich der Temperatursensor derart unmittelbar hinter der Verdunstungskammer, dass eine Temperaturerfassung noch innerhalb des Dosierkanals, also bevor der den Dosierkanal durchströmende nun mit Narkosemittel angereicherte Teilgasstrom eventuell mit dem den Frischgaskanal durchströmenden Teilstrom wieder vermischt wird. Der Temperatursensor erfasst somit auf vorteilhafte Weise die Temperatur des die Verdunstungskammer verlassenden mit Narkosemittel angereicherten Gas- oder Teilgasstroms.

Auf bevorzugte Weise erfolgt die Ansteuerung des Heizelementes durch die Steuereinheit in Abhängigkeit der mit dem Temperatursensor erfassten Temperaturwerte und einer für das jeweils verwendete Narkosemittel spezifischen Dampfdruckkurve. Anhand der vom Anästhesisten vorgegebenen Soll-Konzentration von Narkosemittel im Gasstrom wird somit das Heizelement unter Berücksichtigung der in der Steuerung hinterlegten Dampfdruckkurve des jeweils verwendeten Narkosemittels bedarfsgerecht angesteuert. Durch das Vorsehen eines Temperatursensors hinter der Verdunstungskammer wird hierbei ein geschlossener Regelkreis erzeugt, wobei in Abhängigkeit der gemessenen Temperatur, der gewünschten Soll-Konzentration an Narkosemittel und den thermodynamischen Eigenschaften des jeweils verwendeten Narkosemittels über die Steuereinheit die Heizleistung des Heizelementes eingestellt und bedarfsgerecht, insbesondere in Abhängigkeit des Operationsfortschritts und somit der gewünschten Narkosetiefe, angepasst werden kann.

In einer besonders speziellen Ausführungsform der Erfindung weist die Verdunstungskammer wenigstens zwei Bereiche auf, die bevorzugt in Strömungsrichtung in Reihe hintereinander angeordnet sind. Vorzugsweise sind die wenigstens zwei Bereiche derart ausgeführt, dass, eine Verdunstung zumindest nahezu vollständig in einem ersten Bereich stattfindet, während in einem zweiten Bereich eine Vergleichmäßigung der Temperatur- sowie der Konzentrationsverteilung innerhalb des Gasstroms erreicht wird.

Eine weitere vorteilhafte Gestaltung der Verdunstungskammer sieht vor, dass eine mittlere Oberflächentemperatur in der Verdunstungskammer während des Betriebs einen Wert in einem Bereich zwischen 0 und 40 °C, insbesondere zwischen 30 und 40°C nicht übersteigt. Besonders geeignet erscheint eine Ausführungsform, bei der die mittlere Oberflächentemperatur in der Verdunstungskammer während des Betriebs unterhalb eines Wertes von 35°C bleibt.

Zusätzlich zu einer Vorrichtung betrifft die Erfindung auch ein Verfahren zur Dosierung eines Narkosemittels in einen Gasstrom, bei dem über einen Gaseinlass ein mit Narkosemittel anzureicherndes Gas von einer Atemgas- bzw. Frischgasquelle kommend erwärmt und einer Verdunstungskammer zugeleitet wird, in der dem Gas ein Narkosemittel zudosiert wird und bei dem ein mit dem Narkosemittel angereichertes Gas über einen Gasauslass einem Patientenanschluss zugeleitet wird. Das erfindungsgemäße Verfahren zeichnet sich dadurch aus, dass das mit dem Narkosemittel anzureichernde Gas derart erwärmt wird bevor es in die Verdunstungskammer gelangt, dass in der Verdunstungskammer eine Menge des Narkosemittels in Abhängigkeit wenigstens eines Zustandsparameters des mit dem Narkosemittel anzureichernden und/oder des zumindest teilweise mit dem Narkosemittel angereicherten Gases zugeführt wird.

Wesentlich ist somit, dass der Gasstrom des mit Narkosemittel anzureichernden Gases noch vor Eintritt in die Verdunstungskammer unter Berücksichtigung einer Führungsgröße, also eines Sollwerts für einen ausgewählten Zustandsparameter des vor, in und/oder hinter der Verdunstungskammer befindlichen Gasstroms erwärmt wird. Bei einem derartigen Sollwert handelt es sich bevorzugt um eine Gastemperatur und/oder eine stoffliche Zusammensetzung des Gasstroms.

In einer speziellen Ausführungsform erfolgt die Erwärmung des mit Narkosemittel anzureichernden Gasstroms vor Eintritt in die Verdunstungskammer in Abhängigkeit einer nach Verlassen der Verdunstungskammer benötigten Soll-Konzentration des Narkosemittels und/oder eines benötigten Narkosemittelvolumenstroms, also der erforderlichen Narkosemittelmenge. Auf bevorzugte Weise wird hierbei genau die Menge von Narkosemittel in der Verdunstungskammer verdunstet und dem Gasstrom zugemischt, dass die gewünschte Soll-Konzentration und/oder der erforderliche Narkosemittelvolumenstrom erreicht wird.

Vorzugsweise erfolgt das Verdunsten des Narkosemittels unter den Bedingungen einer adiabaten Oberfläche in der Verdunstungskammer, insbesondere in einem Verdunstungsbereich der Verdunstungskammer.

In einer speziellen Weiterbildung der Erfindung weist die Verdunstungskammer wenigstens zwei in Strömungsrichtung hintereinander angeordnete Bereiche auf, wobei in einem ersten Bereich zumindest nahezu vollständig die Verdunstung der zur Anreicherung des Gasstroms erforderlichen Narkosemittelmenge erfolgt, während der zweite Bereich fast ausschließlich der Vergleichmäßigung der Temperatur des Gasstroms sowie der Mischung des Narkosemittels mit dem Gasstrom dient. Temperatur- und/oder Narkosemittel-Konzentrationsgradienten innerhalb des die Verdunstungskammer verlassenden Gasstroms werden auf diese Weise zumindest weitgehend vermieden.

Auf vorteilhafte Weise findet hinter der Verdunstungskammer eine Messung der Temperatur des mit dem Narkosemittel angereicherten Teilgasstroms oder des mit dem Narkosemittel angereicherten Gasstroms statt, wobei die erfassten Temperaturwerte der Ansteuerung des Heizelementes zugrunde gelegt werden und so ein geschlossener Regelkreis erzeugt wird. Die Temperatur des Teilgasstroms oder des Gasstroms hinter der Verdunstungskammer stellt hierbei auf bevorzugte Weise eine Regelgröße dar, die durch gezielte Ansteuerung der Heizleistung des Heizelementes als Stellgröße erreicht werden soll. Die Regelgröße wird hierbei in Abhängigkeit der jeweils gewünschten Soll-Konzentration des Narkosemittels sowie des Volumenstroms ausgewählt.

Im Folgenden wird die Erfindung ohne Beschränkung des allgemeinen Erfindungsgedankens anhand von Ausführungsbeispielen unter Bezugnahme auf die Figuren näher erläutert. Dabei zeigen:
- Fig. 1:: Blockschaltbild des erfindungsgemäß ausgeführten Narkosemitteldosierverfahrens;
- Fig. 2:: Dampfdruckkurven unterschiedlicher volatiler Narkosemittel;
- Fig. 3:: grafische Darstellung der bei unterschiedlichen Einsatzbedingungen benötigten Heizleistungen eines erfindungsgemäß angeordneten Heizelements sowie
- Fig. 4:: grafische Darstellung der Energiebilanz in Bezug auf die Verdunstungskammer.

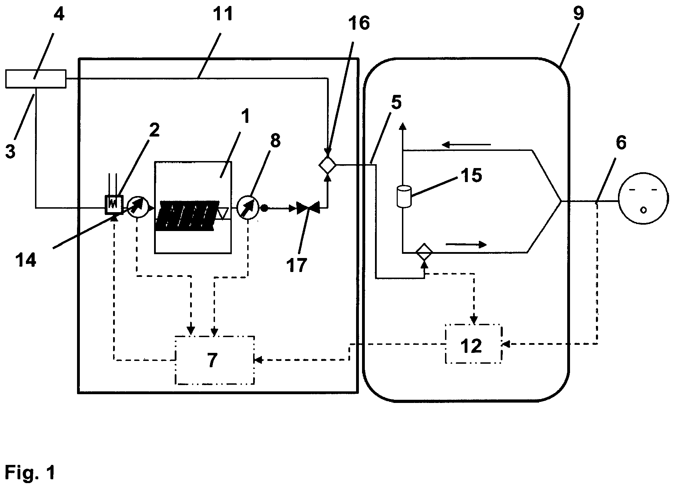

Figur 1 zeigt ein Blockschaltbild eines erfindungsgemäß ausgeführten Narkosemitteldosierers. Ein derartiger Narkosemitteldosierer ist in wird mit einer Anästhesiemaschine kombiniert, deren Ventilatoreinheit 15 einen mit dem volatilen Narkosemittel angereicherten Gasstrom als Atemgasstrom über ein Patientenanschlussstück 6 für einen Patienten zur Verfügung stellt. Die Ventilatoreinheit 15 wird mit Hilfe einer Ventilatorsteuereinheit angesteuert.

Von einer Frischgasquelle 4 kommend gelangt das Frischgas, das mit Narkosemittel angereichert werden soll, zunächst über einen Gaseinlass 3 in die Einheit zur Dosierung von Narkosemittel. Strömungstechnisch parallel zu dem Dosierkanal 13, in dem sich ein Heizelement 2 und eine Verdunstungskammer 1 befinden, ist ein Frischgaskanal 11 angeordnet. Somit wird ein erster Teilgasstrom in den Dosierkanal 13 geleitet während ein zweiter Teilgasstrom das Heizelement 2 und die Verdunstungskammer 1 umströmt. In einem Mischpunkt 16 werden der den Frischgaskanal 11 sowie der den Dosierkanal 13 verlassende Teilgasstrom vor Erreichen des Patientenanschlussstücks 6 gemischt.

Gemäß dem in Figur 1 dargestellten Ausführungsbeispiel erfolgt die Aufteilung des mit Narkosemittel anzureichernden Frischgasstroms in einen ersten und einen zweiten Teilgasstrom innerhalb der Anästhesiemaschine derart, dass ein erster Teilgasstrom, der mit Narkosemittel angereichert werden soll, und ein zweiter Teilgasstrom, der das Heizelement 2 und die Verdunstungskammer umströmt, gebildet werden. In Bezug auf die Anordnung des Heizelements 2 ist es wahlweise möglich, dieses in eine Baueinheit gemeinsam mit der Verdunstungskammer 1 oder in die Anästhesiemaschine zu integrieren. Insbesondere sofern die Verdunstungskammer 1 einschließlich ihres Narkosemitteltanks von der Anästhesiemaschine abnehmbar ausgeführt ist, ist es von Vorteil, wenn das Heizelement 2 in der Anästhesiemaschine angeordnet ist.

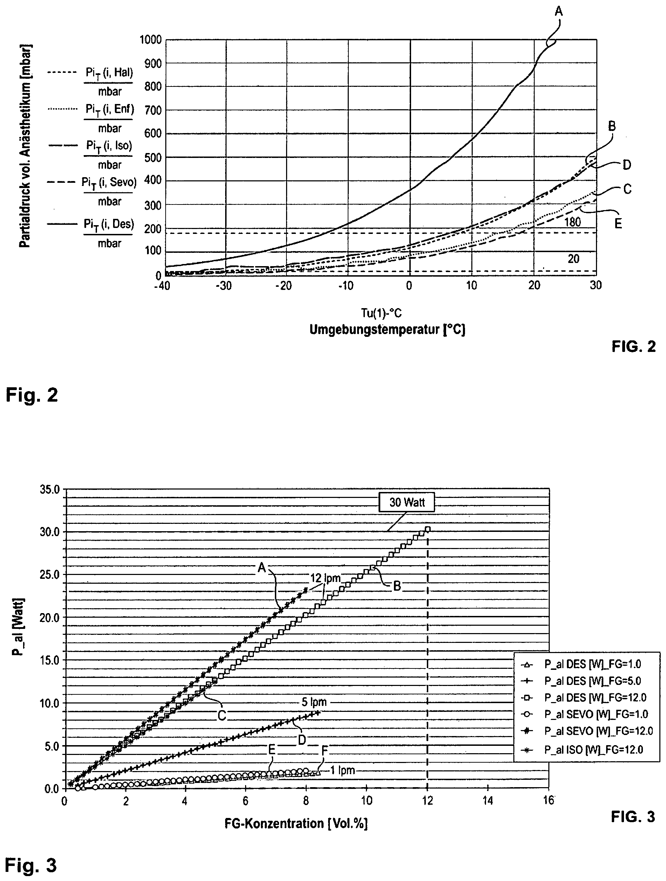

In Figur 1 befindet sich innerhalb des von einer punktlinierten Linie umgebenden Bereichs der Dosierkanal 13, der im Folgenden näher erläutert werden wird. Der den Dosierkanal 13 durchströmende Teilgasstrom wird zunächst einem Heizelement 2 zugeführt. In diesem Heizelement 2 wird der Teilgasstrom bedarfsgerecht erwärmt und anschließend der Verdunstungskammer 1 zugeführt. Innerhalb der Verdunstungskammer 1 befindet sich in einem Tank ein flüssiges Narkosemittel. Grundsätzlich kommen als Narkosemittel u.a. Halothan, Enfluran, Isofluran, Sevofluran und Desfluran in Frage, wobei bei der Verwendung von Desfluran zu beachten ist, dass dieses Narkosemittel unter Normaldruck bei Raumtemperatur gasförmig ist und daher druckbeaufschlagt bevorratet werden sollte. Die Dampfdruckkurven der zuvor genannten Narkosemittel sind Figur 2 zu entnehmen.

Das Gas, das in die Verdunstungskammer 1 einströmt, gelangt in einen Verdunstungsbereich, in dem zumindest ein Teil des Narkosemittels verdunstet und so dem Teilgasstrom zugemischt wird. Hierbei ist die Verdunstungskammer 1 auf bevorzugte Weise derart ausgeführt, dass der Tank und der Verdunstungsbereich ein Bauteil bilden, das über Befestigungselemente zur lösbaren Befestigung dieses Narkosemittelverdampfers an der Anästhesiemaschine verfügt.

Der in die Verdunstungskammer eintretende erwärmte Gasstrom gibt hier seine Wärme zumindest teilweise an das flüssige Narkosemittel ab, das daraufhin entsprechend des Wärmeeintrags, der vom Gasvolumenstrom und der zwischen der Gasstromtemperatur und der Narkosemitteltemperatur herrschenden Temperaturdifferenz abhängt, verdunstet. Um zu gewährleisten, dass die benötigte Menge von Narkosemittel verdunstet und so schließlich die entsprechende Soll-Konzentration an Narkosemittel bzw. der benötigte Narkosemittelvolumenstrom in dem Gasstrom enthalten ist, der dem Patienten zugeleitet wird, wird das Heizelement 2 vor der Verdunstungskammer 1 derart angesteuert, dass die richtige Menge von Narkosemittel innerhalb der Verdunstungskammer 1 verdunstet. Das mit dem Narkosemittel angereicherte Gas verlässt die Verdunstungskammer 1 über eine Austrittsleitung, in der sich ein Temperatursensor 8 zur Erfassung der Austrittstemperatur des mit Narkosemittel angereicherten Gases befindet. Auf vorteilhafte Weise befindet sich in diesem Bereich ebenfalls ein Absperrventil 17, so dass für den Fall, in dem kein Narkosemittel benötigt wird, ein Austritt von Narkosemittel aus der Verdunstungskammer zuverlässig verhindert wird.

Die von dem hinter der Verdunstungskammer 1 befindlichen Temperatursensor 8 erfassten Austrittstemperaturwerte werden ebenfalls an eine Steuereinheit 7 übermittelt und schließlich der Ansteuerung des Heizelementes 2 zugrunde gelegt. Das Heizelement 2 wird hierbei derart angesteuert, dass einerseits Auskühlungseffekte der den Verdunstungsbereich der Verdunstungskammer 1 umgebenden Bauteile ausgeglichen werden und andererseits die erforderliche Menge an Narkosemittel innerhalb der Verdunstungskammer 1 verdunstet und dem Gasstrom zugemischt wird. Ergänzend sind hierzu in der Steuereinheit 7 die thermodynamischen Eigenschaften, insbesondere die Dampfdruckkurve, des jeweils zum Einsatz kommenden Narkosemittels hinterlegt. Die abgespeicherten Dampfdruckkurven repräsentieren jeweils den Druck, bei dem sich in einem abgeschlossenen System ein Dampf mit der zugehörigen, flüssigen Phase des verwendeten Narkosemittels im thermodynamischen Gleichgewicht befindet. Der Dampfdruck nimmt mit steigender Temperatur zu und ist als stoffspezifische Größe abhängig vom jeweils zum Einsatz kommenden Narkosemittel. Dampfdruckkurven unterschiedlicher für eine Nutzung in Frage kommender Narkosemittel sind der Figur 2 zu entnehmen.

Schließlich erreicht der mit Narkosemittel angereicherte Teilgasstrom den Mischpunkt 16, in dem er wiederum mit dem den Frischgaskanal 11 verlassenden Teilgasstrom gemischt wird, so dass beide Teilgasströme im Anschluss hieran als ein Gasstrom über das Patientenanschlussstück 6 dem Patienten zugeleitet werden. Der am Mischpunkt 16 einströmende Teilgasstrom, der den Frischgaskanal 11 verlässt, enthält kein Narkosemittel. Der Teilgasvolumenstrom, der den Dosierkanal 13 durchströmt und die auf der Grundlage der erfolgten Temperaturmessung am Ausgang der Verdunstungskammer 1 ermittelte Narkosemittelkonzentration werden mit der spezifischen Verdampfungsenthalpie des verwendeten Narkosemittels multipliziert, um so die Heizleistung für das Heizelement 2 zu bestimmen. Hierbei erfolgt die Zudosierung von Narkosemittel in jedem Fall in der Verdunstungskammer 1 derart, dass der Gasstrom, der dem Patienten über ein Patientenanschlussstück 6 zugeleitet wird, die vom Arzt vorgegebene Soll-Konzentration und/oder den vorgegebenen Narkosemittelvolumenstrom aufweist.

Die zentrale Steuereinheit 7 des Narkosemitteldosierers verfügt über ein User-Interface, das datentechnisch an diese angebunden ist und über das der Nutzer die benötigten Parameter, insbesondere das verwendete Narkosemittel sowie die benötigte Soll-Konzentration und/oder den erforderlichen Volumenstrom des Narkosemittels eingeben kann. In der Steuereinheit 7 wird unter Zugrundelegung der erfassten Austrittstemperaturen des Gases und der narkosemittelspezifischen Dampfdruckkurve die Konzentration des Narkosemittels im Dosierkanal 13 hinter der Verdunstungskammer 1 ermittelt. In Abhängigkeit der hier ermittelten Konzentration und/oder der mit dem Gasstrom mitgeführten Narkosemittelmenge sowie der in die Steuereinheit 7 vom Nutzer eingegebenen Soll-Konzentration bzw. SollVolumenstrom wird der benötigte Gesamtgasvolumenstrom sowie die geeignete Aufteilung in entsprechende Teilgasströme berechnet und ein Steuersignal erzeugt, um mithilfe eines elektronischen Regelventils 10 die entsprechende Aufteilung in Teilgasströme zu realisieren. Bei der zuvor beschriebenen technischen Lösung wird die am Patientenanschlussstück 6 benötigte Soll-Konzentration von Narkosemittel und/oder der erforderliche Narkosemittelvolumenstrom im Gasstrom von dem Anwender über das User-Interface eingegeben und die entsprechenden Werte datentechnisch in der Steuereinheit 7 hinterlegt. Ebenso ist es allerdings denkbar, dass die Soll-Konzentration bzw. der Sollvolumenstrom nicht vom Anwender eingegeben wird, sondern anhand von operations- und/oder patientenspezifischen Parametern ermittelt und daraufhin der zentralen Steuereinheit 7 des Narkosemitteldosierers zur Verfügung gestellt wird. In diesem Fall ist eine vollautomatische Narkosemittelkonzentrationsregelung realisierbar.

Im Folgenden wird beispielhaft anhand spezieller Zahlenwerte die Aufteilung des Frischgasstroms in einen ersten, den Dosierkanal 13 durchströmenden Teilgasstrom und einen zweiten, den parallel dazu angeordneten Frischgaskanal 11 durchströmenden Teilgasstrom erläutert.

Mit Hilfe der in Figur 2 dargestellten und in der zentralen Steuerung hinterlegten Dampfdruckkurven kann zunächst der Partialdruck des zum Einsatz kommenden Narkosemittels für die in der Verdunstungskammer herrschende Temperatur ermittelt werden. Bei einer Temperatur von 20°C ergibt sich hierbei beispielsweise für Sevoflurane ein Partialdruck von 200 mbar bzw. 200 hPa. Weiterhin wird auf der Grundlage der über eine Nutzerschnittstelle, insbesondere über einen Touchscreen oder eine Tastatur, eingegebenen Sollkonzentration des Narkosemittels im Frischgasstrom, der dem Patienten zugeleitet werden soll, der benötigte Narkosemittelpartialdruck ermittelt. Bei einer gewünschten Narkosemittelkonzentration von 3 Vol% ergibt sich bei einem Gesamtdruck von 1000 mbar bzw. 1000 hPa zum Beispiel ein Narkosemittelpartialdruck von 30 mbar. Gemäß des hier gewählten speziellen Zahlenbeispiels bedeutet dies bei einem dem Patienten zugeleiteten Frischgasvolumenstrom von 5 l/min für die Aufteilung der Teilgasströme auf den Dosier- 13 bzw. den Frischgaskanal 11, dass ein erster Teilvolumenstrom von 0,751 l/min den Dosierkanal 13 durchströmt, wo dieser mit der erforderlichen Menge von Narkosemittel angereichert wird, und ein zweiter Teilvolumenstrom von 4,249 l/min den Frischgaskanal 11 durchströmt.

In Figur 2 sind die Dampfdruckkurven der zurzeit hauptsächlich für eine Verwendung in Frage kommenden Narkosemittel dargestellt. So werden derzeit in der Anästhesie als Narkosemittel leicht flüchtige Flüssigkeiten und Gase wie Isofluran, Sevofluran und Desfluran eingesetzt. Derartige Inhalationsanästhetika sorgen einerseits für weitgehende Schmerzfreiheit, entspannen die Muskulatur und führen andererseits zu einem Bewusstseinsverlust und dämpfen die Schutzreflexe. Grundsätzlich ist das erfindungsgemäße Verfahren auch für den Einsatz von Halothan geeignet, das allerdings in Europa und den USA weitgehend von den drei vorgenannten Inhalationsanästhetika abgelöst worden ist.

Ein weiteres grundsätzlich für eine Verwendung in Frage kommendes Narkosemittel ist Enfluran, bei dem es sich um ein dem Halothan ähnliches volatiles Narkosemittel aus der Gruppe der Flurane handelt. Dieses Narkosemittel führt ebenfalls zu einem Bewusstseinsverlust und wirkt muskelrelaxierend, führt jedoch nur zu einer vergleichsweise geringen Schmerzunterdrückung und wird daher nur noch sehr selten verwendet.

In diesem Zusammenhang sind in Figur 3 übereinander die Dampfdruckkurven von Desfluran (A), Halothan (B), Enfluran (C), Isofluran (D) sowie Sevofluran (E) in einem Temperaturbereich von -40° C bis +30°C und einem Druckbereich zwischen 0 mbar und 1000 hPa aufgeführt. Hierbei zeichnet sich Desfluran dadurch aus, dass es bei Raumtemperatur und Umgebungsdruck in den gasförmigen Zustand übergeht. Aus diesem Grund wird Desfluran (A) üblicherweise druckbeaufschlagt und somit in flüssigem Aggregatzustand bevorratet.

Figur 3 zeigt beispielhaft verschiedene Heizleistungen für unterschiedliche Betriebszustände der erfindungsgemäß ausgeführten Narkosemittelverdunstung in Bezug auf die Verwendung unterschiedlicher Narkosemittel und die Einstellung verschiedener Soll-Konzentrationen. Hierbei geht in die Heizleistungsberechnung innerhalb der Steuereinheit wenigstens ein Korrekturfaktor ein, der von der konstruktiven Auslegung der Verdunstungskammer 1, insbesondere des Verdunstungsbereiches, und der technischen Ausführung des Heizelementes 2 abhängt. Somit beeinflussen der thermische Wirkungsgrad des Heizelementes 2 sowie des Verdunstungsbereiches der Verdunstungskammer 1 direkt den thermischen Wirkungsgrad des Narkosemittelverdunsters.

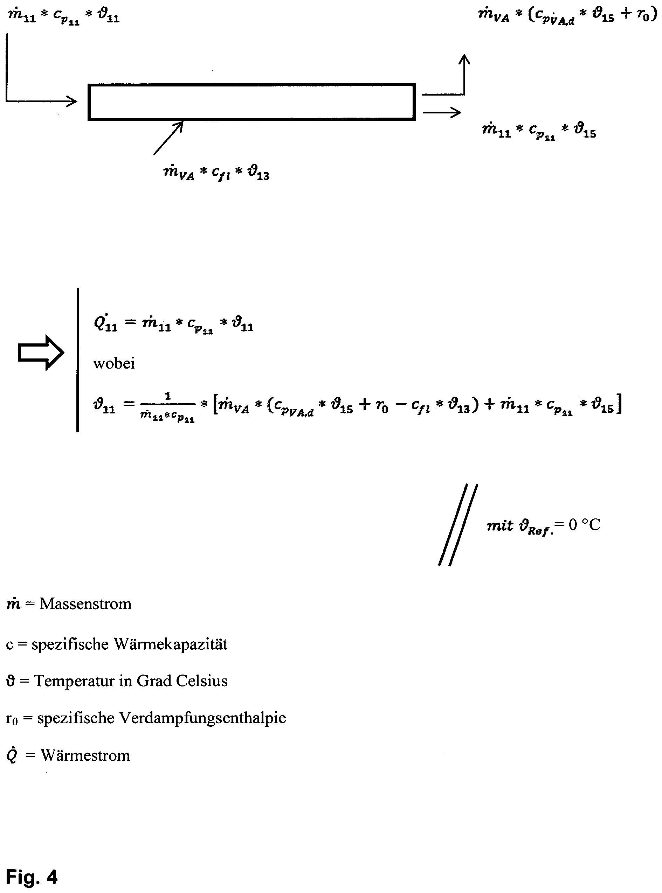

In Figur 4 ist die Energiebilanz der Verdunstungskammer 1 grafisch dargestellt, wobei die Verdunstungskammer 1 thermodynamisch als Wärmeübertrager aufgefasst wird.

Über den im Heizelement 2 erwärmten Frischgasstrom bzw. Frischgasteilstrom wird der Verdunstungskammer 1 eine zur Verdunstung des Narkosemittels nutzbare Wärme zugeführt, die vom Gasvolumen- bzw. Gasmassenstrom, der spezifischen Wärmekapazität und der Temperaturdifferenz zwischen der Temperatur des eintretenden Gasstroms und der Temperatur des Narkosemittels in der Verdunstungskammer 1 abhängt. Diese Wärme wird zur Verdunstung der jeweils benötigten Narkosemittelmenge genutzt, so dass die Temperatur des Gasstroms entsprechend der narkosemittelspezifischen Verdampfungsenthalpie wieder abkühlt. Der die Verdunstungskammer 1 verlassende Gasstrom kann thermodynamisch nun als Gasstrom, der sich einerseits aus einem Frischgasstrom und andererseits aus einem Narkosemittelstrom zusammensetzt, angesehen werden. Ausgehend von dieser Überlegung kann für den mit Narkosemittel angereicherten Gasstrom eine Wärmebilanz aufgestellt werden, die einerseits den Massenstrom des Frischgases, dessen spezifische Wärmekapazität sowie die Temperaturdifferenz zwischen der Temperatur des in die Verdunstungskammer 1 einströmenden und der Temperatur des aus der Verdunstungskammer 1 ausströmenden Gasstroms und andererseits den Massenstrom des verdampften Narkosemittels, dessen spezifische Wärmekapazität und wiederum die Temperaturdifferenz des Gasstroms berücksichtigt. Auf der Grundlage dieser Wärmebilanzierung kann unter Berücksichtigung der benötigten Soll-Konzentration des verwendeten Narkosemittels oder des benötigten Narkosemittelvolumenstroms, der hinter der Verdunstungskammer 1 erfassten Temperatur und der Dampfdruckkurve des Narkosemittels die Wärme ermittelt werde, die benötigt wird, um die benötigte Narkosemittelmenge zu verdampfen. Unter Berücksichtigung des thermischen Wirkungsgrads der Verdunstungskammer 1 und des Wirkungsgrads des Heizelements 2 ergibt sich hieraus die benötigte Heizleistung die über die zentrale Steuerung beim Heizelement 2 abgefordert wird.

Wesentlich an der erfindungsgemäßen technischen Lösung ist, dass der Narkosemitteldosierer weder am Eintritt noch am Austritt eine Messung der Narkosemittelkonzentration benötigt, da die Konzentration am Austritt auf der Grundlage der Dampfdruckkurve und der Verdunstungskammerauslegung ermittelt werden kann.

### Bezugszeichenliste

- 1: Verdunstungskammer
- 2: Heizelement
- 3: Gaseinlass
- 4: Gasquelle
- 5: Gasauslass
- 6: Patientenanschlussstück
- 7: Zentrale Steuereinheit
- 8: Temperatursensor hinter Verdunstungskammer
- 9: Anästhesiekreislauf
- 10: Absperrventil
- 11: Frischgaskanal
- 12: Ventilatorsteuereinheit
- 13: Dosierkanal
- 14: Temperatursensor vor Verdunstungskammer
- 15: Ventilatoreinheit
- 16: Mischpunkt
- 17: Absperrventil

## Patentansprüche

1. Vorrichtung zur Dosierung eines Narkosemittels in einen Gasstrom mit einem Gaseinlass (3), über den mit dem Narkosemittel anzureicherndes Gas von einer Gasquelle (4) kommend einströmt und einem Gasauslass (5), über den mit dem Narkosemittel angereichertes Gas zu einem Patientenanschluss (6) ausströmt, wobei in Strömungsrichtung des Gases zwischen dem Gaseinlass (3) und dem Gasauslass (5) eine Verdunstungskammer (1), in der dem Gasstrom zumindest zeitweise ein Narkosemittel zudosierbar ist und die einen Tank für flüssiges Narkosemittel und einen Verdunstungsbereich, in dem flüssiges Narkosemittel wenigstens teilweise verdunstet, aufweist, und ein Heizelement (2) zur zumindest zeitweisen Erwärmung des Gases angeordnet sind, wobei das Heizelement (2) in Strömungsrichtung des Gases vor derVerdunstungskammer (1) angeordnet ist,
**dadurch gekennzeichnet, dass** das Heizelement (2) von einer Steuereinheit (7) derart angesteuert wird, dass dem mit Narkosemittel anzureichernden Gas Warme zur Verdunstung des in der Verdunstungskammer (1) befindlichen Narkosemittels in Abhängigkeit wenigstens eines Zustandsparameters des mit dem Narkosemittel anzureichernden und/oder des zumindest teilweise mit dem Narkosemittel angereicherten Gases sowie in Abhängigkeit einer nach Verlassen der Verdunstungskammer (1) benötigten Narkosemittelmenge und/oder einer nach Verlassen der Verdunstungskammer (1) benötigten Sollkonzentration des Narkosemittels im Gas und/oder eines erforderlichen Narkosemittelvolumenstroms zugeführt wird.

2. Vorrichtung nach Anspruch 1,
**dadurch gekennzeichnet, dass** die Verdunstungskammer (1) und der Tank als ein Bauteil ausgeführt sind.

3. Vorrichtung nach einem der Ansprüche 1 oder 2, **dadurch gekennzeichnet, dass** diese über wenigstens ein Befestigungselement zur lösbaren Verbindung mit einem Anästhesiegerät verfügt.

4. Vorrichtung nach einem der Ansprüche 1 bis 3,
**dadurch gekennzeichnet, dass** das Heizelement (2) derart angesteuert wird, dass eine Temperatur einer die Verdunstungskammer (1) nach Innen begrenzenden Kammerwand während des Betriebs zumindest nahezu konstant ist.

5. Vorrichtung nach einem der Ansprüche 1 bis 4,
**dadurch gekennzeichnet, dass** sich in Strömungsrichtung hinter der Verdunstungskammer (1) ein Temperatursensor (8) zur Erfassung einer Temperatur des mit Narkosemittel angereicherten Gases befindet, der über eine Datenstrecke mit der Steuereinheit (7) verbunden ist.

6. Vorrichtung nach Anspruch 5,
**dadurch gekennzeichnet, dass** das Heizelement (2) von der Steuereinheit (7) in Abhängigkeit der mit dem Temperatursensor (8) erfassten Temperaturwerte und einer für das Narkosemittel spezifischen Dampfdruckkurve angesteuert wird.

7. Vorrichtung nach einem der Ansprüche 1 bis 6,
**dadurch gekennzeichnet, dass** die Verdunstungskammer (1) wenigstens zwei Bereiche aufweist, wobei eine Narkosemittelverdunstung zumindest nahezu ausschließlich in einem dieser Bereiche erfolgt.

8. Vorrichtung nach einem der Ansprüche 1 bis 7,
**dadurch gekennzeichnet, dass** das Heizelement (2) derart angesteuert wird, dass der die Verdunstungskammer (1) verlassende Gasstrom zumindest nahezu mit Narkosemittel gesättigt ist.

9. Vorrichtung nach einem der Ansprüche 1 bis 8,
**dadurch gekennzeichnet, dass** das Heizelement (2) und die Verdunstungskammer (1) konstruktiv in ein Bauelement integriert sind.

10. Vorrichtung nach Anspruch 9,
**dadurch gekennzeichnet, dass** eine Außenwand der Verdunstungskammer (1) derart geformt ist, dass das Heizelement (2) innerhalb einer von der Außenwand gebildeten halboffenen Ausnehmung aufgenommen wird.

11. Verfahren zur Dosierung eines Narkosemittels in einen Gasstrom, bei dem über einen Gaseinlass (3) ein mit dem Narkosemittel anzureicherndes Gas von einer Gasquelle (4) kommend erwärmt, und einer Verdunstungskammer (1), die einen Tank für flüssiges Narkosemittel und einen Verdunstungsbereich, in dem flüssiges Narkosemittel wenigstens teilweise verdunstet, aufweist, zugeleitet wird, in der dem Gas ein Narkosemittel zudosiert wird und bei dem ein mit dem Narkosemittel angereichertes Gas über einen Gasauslass (5) einem Patientenanschluss (6) zugeleitet wird, wobei das mit dem Narkosemittel anzureichernde Gas erwärmt wird bevor es in die Verdunstungskammer (1) gelangt,
**dadurch gekennzeichnet, dass** das mit dem Narkosemittel anzureichernde Gas derart erwärmt wird, dass in der Verdunstungskammer (1) eine Menge des Narkosemittels in Abhängigkeit wenigstens eines Zustandsparameters des mit dem Narkosemittel anzureichernden und/oder des zumindest teilweise mit dem Narkosemittel angereicherten Gases sowie in Abhängigkeit einer nach Verlassen der Verdunstungskammer (1) benötigten Narkosemittelmenge und/oder einer Sollkonzentration des Narkosemittels und/oder eines Narkosemittelvolumenstroms in dem die Verdunstungskammer (1) verlassenden Gasstrom, zugemischt wird.

12. Verfahren nach Anspruch 11, **dadurch gekennzeichnet, dass** das mit dem Narkosemittel anzureichernde Gas derart erwärmt wird, dass eine Verdunstung des Narkosemittels in der Verdunstungskammer (1) unter zumindest nahezu adiabaten Verhältnissen erfolgt.

13. Verfahren nach einem der Ansprüche 11 oder 12, **dadurch gekennzeichnet, dass** eine Erwärmung des mit Narkosemittel anzureicherndes Gases derart erfolgt, dass während eines Betriebs eine mittlere Oberflächentemperatur in der Verdunstungskammer in einem Bereich zwischen 0 und 40°C liegt.

14. Verfahren nach einem der Ansprüche 11 bis 13,
**dadurch gekennzeichnet, dass** der mit dem Narkosemittel anzureichernde Gasstrom derart vor Eintritt in die Verdunstungskammer (1) erwärmt wird, dass der die Verdunstungskammer (1) verlassende Gasstrom zumindest nahezu mit Narkosemittel gesättigt ist.

15. Verfahren nach einem der Ansprüche 11 bis 14
**dadurch gekennzeichnet, dass** wenigstens eine Temperatur des mit Narkosemittel anzureichernden Gaststroms vor der Verdunstungskammer (1) und/oder eine Temperatur des mit Narkosemittel angereicherten Gasstroms hinter der Verdunstungskammer (1) erfasst wird.

## Claims

1. A device for dosing an anaesthetic into a gas stream having a gas inlet (3), by way of which gas that is to be enriched with the anaesthetic flows in, coming from a gas source (4), and a gas outlet (5), by way of which gas enriched with the anaesthetic flows out to a patient connection (6), wherein arranged in the direction of flow of the gas between the gas inlet (3) and the gas outlet (5) there is an evaporation chamber (1), in which an anaesthetic can be dosed into the gas stream at least occasionally and which has a tank for liquid anaesthetic and an evaporation region in which liquid anaesthetic evaporates at least in part, and there is a heating element (2) for at least occasionally heating the gas, wherein the heating element (2) is arranged upstream of the evaporation chamber (1) in the direction of flow of the gas,
**characterised in that** the heating element (2) is activated by a control unit (7) in such a way that heat is supplied to the gas that is to be enriched with anaesthetic in order to evaporate the anaesthetic located in the evaporation chamber (1) as a function of at least one status parameter of the gas to be enriched with the anaesthetic and/or the gas enriched at least in part with the anaesthetic and also as a function of a quantity of anaesthetic required after leaving the evaporation chamber (1) and/or a desired concentration of the anaesthetic in the gas required after leaving the evaporation chamber (1) and/or a necessary anaesthetic volume flow.

2. A device according to claim 1,
**characterised in that** the evaporation chamber (1) and the tank are constructed as one component.

3. A device according to one of claims 1 or 2,
**characterised in that** it is provided with at least one securing element for detachable connection to an anaesthetic apparatus.

4. A device according to one of claims 1 to 3,
**characterised in that** the heating element (2) is activated in such a way that a temperature of a chamber wall, inwardly delimiting the evaporation chamber (1), is at least almost constant during the operation.

5. A device according to one of claims 1 to 4,
**characterised in that** located downstream of the evaporation chamber (1) in the direction of flow there is a temperature sensor (8) for detecting a temperature of the gas enriched with anaesthetic that is connected to the control unit (7) by way of a data link.

6. A device according to claim 5,
**characterised in that** the heating element (2) is activated by the control unit (7) as a function of the temperature values detected by the temperature sensor (8) and a vapour-pressure curve specific to the anaesthetic.

7. A device according to one of claims 1 to 6,
**characterised in that** the evaporation chamber (1) has at least two regions, wherein anaesthetic-evaporation is effected at least almost exclusively in one of these regions.

8. A device according to one of claims 1 to 7,
**characterised in that** the heating element (2) is activated in such a way that the gas stream leaving the evaporation chamber (1) is at least almost saturated with anaesthetic.

9. A device according to one of claims 1 to 8,
**characterised in that** the heating element (2) and the evaporation chamber (1) are integrated structurally into one component.

10. A device according to claim 9,
**characterised in that** an outer wall of the evaporation chamber (1) is shaped in such a way that the heating element (2) is accommodated within a half-open recess formed by the outer wall.

11. A method for dosing an anaesthetic into a gas stream, in which by way of a gas inlet (3) a gas that is to be enriched with the anaesthetic, coming from a gas source (4), is heated and fed to an evaporation chamber (1) that has a tank for liquid anaesthetic and an evaporation region in which liquid anaesthetic evaporates at least in part and in which an anaesthetic is dosed into the gas, and in which a gas that is enriched with the anaesthetic is fed by way of a gas outlet (5) to a patient connection (6), wherein the gas that is to be enriched with the anaesthetic is heated before it reaches the evaporation chamber (1),
**characterised in that** the gas that is to be enriched with the anaesthetic is heated in such a way that in the evaporation chamber (1) a quantity of the anaesthetic is admixed as a function of at least one status parameter of the gas to be enriched with the anaesthetic and/or of the gas enriched at least in part with the anaesthetic and also as a function of a quantity of anaesthetic required after leaving the evaporation chamber (1) and/or a desired concentration of the anaesthetic and/or an anaesthetic volume flow in the gas stream leaving the evaporation chamber (1).

12. A method according to claim 11,
**characterised in that** the gas that is to be enriched with the anaesthetic is heated in such a way that evaporation of the anaesthetic is effected in the evaporation chamber (1) under at least almost adiabatic conditions.

13. A method according to one of claims 11 or 12,
**characterised in that** heating of the gas that is to be enriched with anaesthetic is effected in such a way that during an operation an average surface temperature in the evaporation chamber lies in a range between 0 and 40°C.

14. A method according to one of claims 11 to 13,
**characterised in that** the gas stream that is to be enriched with the anaesthetic is heated in such a way before entry into the evaporation chamber (1) that the gas stream leaving the evaporation chamber (1) is at least almost saturated with anaesthetic.

15. A method according to one of claims 11 to 14,
**characterised in that** at least one temperature of the gas stream that is to be enriched with anaesthetic is detected upstream of the evaporation chamber (1) and/or one temperature of the gas stream enriched with anaesthetic is detected downstream of the evaporation chamber (1).

## Revendications

1. Dispositif dévolu au dosage d'un agent anesthésiant dans un flux gazeux, comprenant une admission (3) de gaz par l'intermédiaire de laquelle du gaz, devant être enrichi en agent anesthésiant, afflue en provenance d'une source (4) de gaz, et une sortie (5) de gaz par l'intermédiaire de laquelle du gaz, enrichi en agent anesthésiant, sort en direction d'un raccord (6) équipant un patient, sachant qu'une chambre de vaporisation (1) dans laquelle un agent anesthésiant peut être au moins temporairement ajouté au flux gazeux de manière dosée, et qui est munie d'une cuve destinée à de l'agent anesthésiant liquide et d'une zone de vaporisation dans laquelle de l'agent anesthésiant liquide est au moins partiellement vaporisé, ainsi qu'un élément chauffant (2) affecté au chauffage au moins temporaire du gaz, sont interposés entre ladite admission (3) de gaz et ladite sortie (5) de gaz dans la direction d'écoulement du gaz, ledit élément chauffant (2) étant implanté devant ladite chambre de vaporisation (1) dans ladite direction d'écoulement du gaz,
**caractérisé par le fait que** l'élément chauffant (2) est piloté par une unité de commande (7), de façon telle que de la chaleur soit délivrée au gaz à enrichir en agent anesthésiant en vue de vaporiser ledit agent anesthésiant, situé dans la chambre de vaporisation (1), en fonction d'au moins un paramètre d'état dudit gaz à enrichir en agent anesthésiant et/ou au moins partiellement enrichi en agent anesthésiant, ainsi qu'en fonction d'une quantité d'agent anesthésiant requise à la sortie de la chambre de vaporisation (1) et/ou d'une concentration de consigne en agent anesthésiant dans le gaz, requise à la sortie de ladite chambre de vaporisation (1), et/ou d'un débit volumique requis dudit agent anesthésiant.

2. Dispositif selon la revendication 1,
**caractérisé par le fait que** la chambre de vaporisation (1) et la cuve sont réalisées sous la forme d'un composant structurel.

3. Dispositif selon l'une des revendications 1 ou 2,
**caractérisé par le fait que** ce dernier est équipé d'au moins un élément de fixation dédié à la liaison amovible avec un appareil d'anesthésie.

4. Dispositif selon l'une des revendications 1 à 3,
**caractérisé par le fait que** l'élément chauffant (2) est piloté de façon telle qu'une température d'une paroi, délimitant la chambre de vaporisation (1) vers l'intérieur, soit au moins approximativement constante au cours du fonctionnement.

5. Dispositif selon l'une des revendications 1 à 4,
**caractérisé par le fait qu'**un capteur de températures (8), conçu pour détecter une température du gaz enrichi en agent anesthésiant et placé derrière la chambre de vaporisation (1) dans la direction de l'écoulement, est connecté à l'unité de commande (7) par l'intermédiaire d'un trajet de données.

6. Dispositif selon la revendication 5,
**caractérisé par le fait que** l'élément chauffant (2) est piloté, par l'unité de commande (7), en fonction des valeurs de température détectées par le capteur de températures (8), et d'une courbe de pression de vapeur spécifique de l'agent anesthésiant.

7. Dispositif selon l'une des revendications 1 à 6,
**caractérisé par le fait que** la chambre de vaporisation (1) comporte au moins deux zones, une vaporisation de l'agent anesthésiant ayant lieu, au moins presque exclusivement, dans l'une de ces zones.

8. Dispositif selon l'une des revendications 1 à 7,
**caractérisé par le fait que** l'élément chauffant (2) est piloté de façon telle que le flux gazeux, quittant la chambre de vaporisation (1), soit au moins approximativement saturé en agent anesthésiant.

9. Dispositif selon l'une des revendications 1 à 8,
**caractérisé par le fait que** l'élément chauffant (2) et la chambre de vaporisation (1) sont structurellement intégrés dans un composant.

10. Dispositif selon la revendication 9,
**caractérisé par le fait qu'**une paroi extérieure de la chambre de vaporisation (1) est configurée de telle sorte que l'élément chauffant (2) soit logé à l'intérieur d'un évidement semi-ouvert, formé par ladite paroi extérieure.

11. Procédé de dosage d'un agent anesthésiant dans un flux gazeux, procédé dans lequel, par l'intermédiaire d'une admission (3) de gaz, un gaz devant être enrichi en agent anesthésiant, provenant d'une source (4) de gaz, est chauffé et délivré à une chambre de vaporisation (1) munie d'une cuve destinée à de l'agent anesthésiant liquide, et d'une zone de vaporisation dans laquelle de l'agent anesthésiant liquide est au moins partiellement vaporisé, chambre dans laquelle un agent anesthésiant est ajouté au gaz, de manière dosée ; et dans lequel, par l'intermédiaire d'une sortie (5) de gaz, un gaz enrichi en agent anesthésiant est délivré à un raccord (6) équipant un patient, sachant que le gaz, devant être enrichi en agent anesthésiant, est chauffé avant de parvenir dans ladite chambre de vaporisation (1),
**caractérisé par le fait que** le gaz, devant être enrichi en agent anesthésiant, est chauffé de telle sorte qu'une quantité d'agent anesthésiant soit ajoutée, dans la chambre de vaporisation (1), en fonction d'au moins un paramètre d'état dudit gaz à enrichir en agent anesthésiant et/ou au moins partiellement enrichi en agent anesthésiant, ainsi qu'en fonction d'une quantité d'agent anesthésiant requise à la sortie de la chambre de vaporisation (1) et/ou d'une concentration de consigne en agent anesthésiant et/ou d'un débit volumique dudit agent anesthésiant dans le flux gazeux quittant ladite chambre de vaporisation (1).

12. Procédé selon la revendication 11,
**caractérisé par le fait que** le gaz, devant être enrichi en agent anesthésiant, est chauffé de telle sorte qu'une vaporisation de l'agent anesthésiant ait lieu, dans la chambre de vaporisation (1), suivant des proportions au moins approximativement adiabatiques.

13. Procédé selon l'une des revendications 11 ou 12,
**caractérisé par le fait qu'**un chauffage du gaz, devant être enrichi en agent anesthésiant, s'opère de façon telle qu'une température moyenne à la surface, dans la chambre de vaporisation, se situe dans une plage comprise entre 0 et 40 °C au cours d'un fonctionnement.

14. Procédé selon l'une des revendications 11 à 13,
**caractérisé par le fait que** le flux gazeux devant être enrichi en agent anesthésiant est chauffé, avant pénétration dans la chambre de vaporisation (1), de façon telle que le flux gazeux, quittant ladite chambre de vaporisation (1), soit au moins approximativement saturé en agent anesthésiant.

15. Procédé selon l'une des revendications 11 à 14,
**caractérisé par** la détection d'au moins une température du flux gazeux devant être enrichi en agent anesthésiant, devant la chambre de vaporisation (1), et/ou d'une température du flux gazeux enrichi en agent anesthésiant, derrière ladite chambre de vaporisation (1).
